(19) Europäisches Patentamt
European Patent Office
Office européen des brevets

(11) Publication number: **0 087 865**
**B1**

(12) **EUROPEAN PATENT SPECIFICATION**

(45) Date of publication of patent specification: **01.10.86**

(21) Application number: **83300622.4**

(22) Date of filing: **08.02.83**

(51) Int. Cl.$^4$: **A 61 K 31/20,** A 61 K 31/23, A 61 K 31/425 // (A61K31/23, 31:20, 31:195),(A61K31/425, 31:23, 31:20, 31:195)

(54) **Pharmaceutical composition.**

(30) Priority: **01.03.82 GB 8205938**

(43) Date of publication of application:
**07.09.83 Bulletin 83/36**

(45) Publication of the grant of the patent:
**01.10.86 Bulletin 86/40**

(84) Designated Contracting States:
**AT BE CH DE FR GB IT LI LU NL SE**

(56) References cited:
**EP-A-0 037 175**
**FR-A-2 231 379**
**GB-A-1 476 624**

**A.L. Lehninger, Biochemistry, 1970, New York, page 521**

(73) Proprietor: **EFAMOL LIMITED**
**71/74 Mark Lane**
**London E.C.3 (GB)**

(72) Inventor: **Horrobin, David Frederick**
**P.O. Box 10**
**Nuns'Island Montreal H3E 1J8 (CA)**

(74) Representative: **Caro, William Egerton et al**
**J. MILLER & CO. Lincoln House 296-302 High Holborn**
**London WC1V 7JH (GB)**

**EP 0 087 865 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the invention

This invention relates to the treatment of certain diseases and disorders primarily, but not exclusively, in the field of human medicine and to compositions for use therein.

General background

Considerable interest has been shown in recent years in the use of prostaglandin (PG) precursors in medicine.

For various reasons when raised levels of prostaglandins are required it is not usually practical to administer naturally occurring prostaglandins such as PGE1 and PGE2 to patients. Consequently, considerable attention has focussed on the use of prostaglandin precursors including linoleic acid, γ-linolenic acid (GLA) and dihomo-γ-linolenic acid (DGLA).

Conversion of these materials in the body is believed to be as shown in the following diagram:—

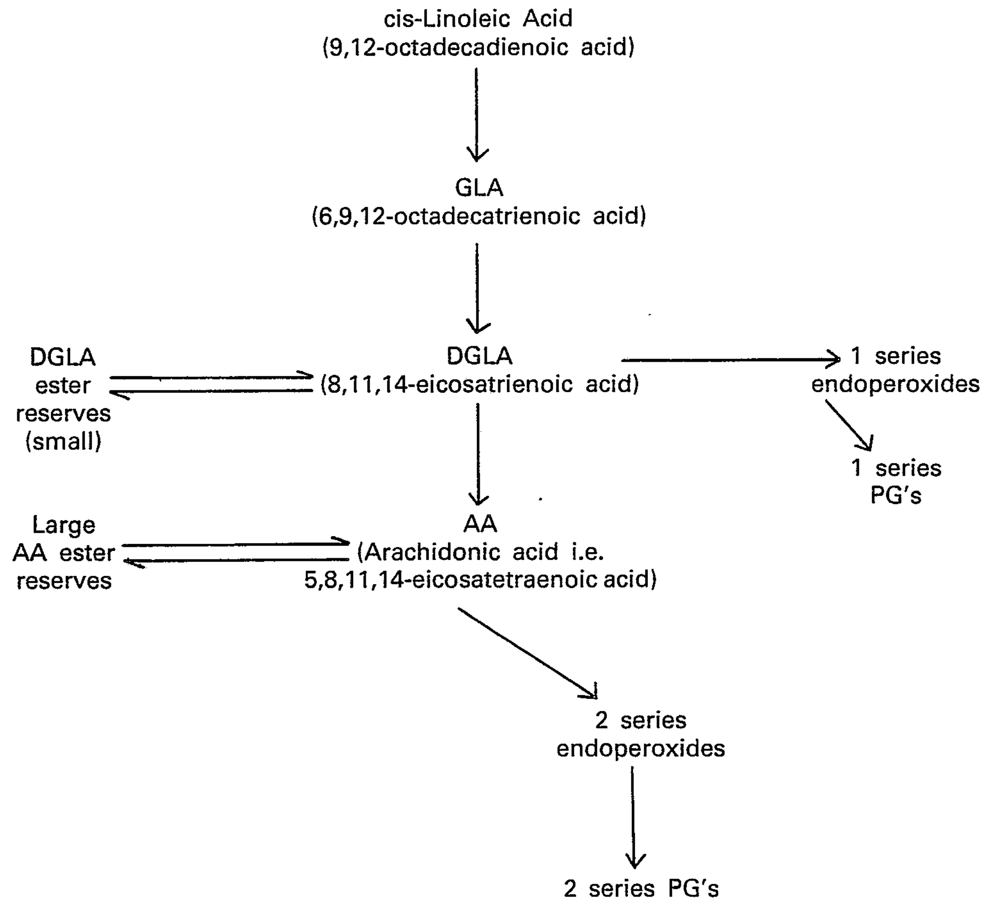


The broad outline of this pathway is well known, and it brings out clearly that a major function of essential fatty acids (EFAs) is to act as precursors for prostaglandins, 1-series PGs being formed from dihomo-γ-linolenic acid (DGLA) and 2-series PGs from arachidonic acid (AA). DGLA and AA are present in food in only small quantities, and the major EFA in food is linoleic acid which is first converted to γ-linolenic acid (GLA) and then to DGLA and AA, the latter step being irreversible. The conversion of linoleic acid to GLA is a limiting step, adequate in the young and healthy body but often inadequate in ageing or in many diseased states.

DGLA is the key substance. GLA is almost completely and very rapidly converted in the body to DGLA and so for practical purposes the oral administration of DGLA and GLA amounts to the same thing. DGLA can be converted to a storage form, changed to arachidonic acid and thence to PGs of the 2-series, or converted to PGs of the 1-series.

A balance between 1-series and 2-series PGs is, the inventor believes, highly significant in terms of overall control of the conversion pathways. Such control is not understood in detail but without restriction to the theory it appears first that PGE2 is able to enhance the formation of 1-series PGs, and second that PGE1 is able to block arachidonic acid mobilisation from tissue stores. Thus the condition for a negative feedback control loop exists; overproduction of PGE2 from AA will activate PGE1 synthesis, the PGE1 will inhibit AA mobilisation, and production of the 2-series PGs will drop. Further, TXA2, an unstable product of

the 2-series endoperoxides arising in 2-series PG production, also appears to limit mobilisation of AA and to enhance 1-series PGs and in particular PGE1 production. Thus again the activity of the 2-series PG synthesis pathway gives rise indirectly to a material that controls that pathway.

EFA Metabolism in cancer cells

One particular characteristic shown by human and animal cancer cells and by transformed cells is a consistent absence of the enzyme delta-6-desaturase which converts linoleic acid to γ-linolenic acid. The inventor believes that this fact is of great significance and that faulty essential fatty acid metabolism is a key factor in cancer.

The outline of the pathways of EFA metabolism in the body is given earlier, and as stated DGLA is the key substance. It can be converted to a storage form, or to PGs of the 1-series, or to arachidonic acid and thence to PGs of the 2-series. The conversion to arachidonic acid is irreversible.

Accordingly it can be seen that since γ-linolenic acid is a necessary precursor of dihomo-γ-linolenic acid and thus of 1-series PGs, and since also cellular stores of DGLA are very limited, cancer cells and transformed cells soon loose the ability to make 1-series PGs and in particular the important compound PGE1.

The inventor believes that many of the characteristic features of transformed and cancer cells are due to this damage to PG metabolism and accordingly that measures used in the treatment of cancer will be more effective, particularly in the long term, if they are supported by measures to restore production of 1-series PGs and particularly PGE1 in the cells.

General medical background

There is increasing evidence that PGs of the 1-series play a vital role in many key areas. First, PGE1 activates T-lymphocytes. Defective T-lymphocytes are believed to be involved in causing a wide range of allergic and inflammatory disorders and in making individuals susceptible to cancer and infections of all types. Second, PGE1 is important in preventing over-production of collagen and fibrous tissue, a factor which plays a major role in arthritis and the so-called collagen diseases. Third PGE1 levels are extremely low in patients with schizophrenia and are moderately low in patients with depression. Fourth, PGE1 appears to be important in controlling cholesterol levels and necessary for the normal actions of insulin. Fifth, PGE1 dilates blood vessels and may be expected to be helpful in any situation in which vessel spasm occurs. Sixth, PGE1 appears to inhibit the production of 2-series PGs, levels of which are raised in a wide variety of inflammatory disorders. Seventh, PGE1 increases production of cyclic AMP which has anti-inflammatory effects.

Glutathione

Glutathione is made up of three aminoacid residues and has formula:

$$\begin{array}{l} HOOC—CH—CH_2—CH_2—CO—NH—CH—CO—NH—CH_2—COOH \\ \qquad\quad | \qquad\qquad\qquad\qquad\qquad\qquad\quad | \\ \qquad\; NH_2 \qquad\qquad\qquad\qquad\qquad\;\; CH_2SH \end{array}$$

It may exist in reduced or oxidised forms and is the most abundant —SH compound in mammalian cells. It has recently come to particular notice in a report by Anna M. vi in Science, *212* 541—542 (1st May 1981). There, remarkable results are given in preventing establishment of aflatoxin-induced tumours in rats and in causing regression and cure of large, established tumours. No explanation is proposed.

Glutathione is also known, for example from the inventor's own book "Prostaglandins: Physiology, Pharmacology and Clinical Significance", Eden Press, Montreal 1978, to be a co-factor able to activate the enzyme PGE isomerase, by which PGEs are formed from 1-series or 2-series endoperoxides.

The invention

The present inventor has considered glutathione afresh in the light of his knowledge of EFA metabolism summarised above and of the above work on glutathione, and has concluded that valuable results can be had from compositions of GLA and/or DGLA, and glutathione, which may be in any convenient form.

Optional further components are any of the agents proposed in the inventor's previous patent applications referred to later herein as enhancing 1-series PG formation or, more broadly expressed, as influencing the 1-series/2-series PG balance in the body in favour of 1-series PGs.

Situations in which the present proposal is of value are set out in detail below. Particular reference is made however to treatment of inflammatory disorders and cancer by use of essential fatty acid/glutathione compositions, and to treatment of cancer with such compositions containing also reverse transformers, in particular thioproline, as described in the inventor's published EP—A—0 037 175.

Details of application generally

The broad aim of the present invention is to influence the 1-series/2-series PG balance in the body in favour of 1-series GP's and specifically to selectively enhance formation of PG's of the 1-series and particularly PGE1. The diseases and disorders below are among those in which such action is indicated:

1. Situations in which defective T-lymphocyte function has been described such as allergic and inflammatory disorders, multiple sclerosis, schizophrenia and cancer.

2. Situations in which regulation of collagen formation and breakdown is defective including rheumatoid arthritis, systemic lupus erythematosus, and various "collagen" diseases.

3. Mental illnesses in which low PGE1 levels have been reported including depression, schizophrenia and alcohol withdrawal and the latter stages of alcohol intoxication. In depression for example platelet PGE1 production is moderately reduced whereas in schizophrenia it is severely reduced.

4. Disorders of lipid and carbohydrate metabolism in particular diabetes mellitus and situations in which blood cholesterol levels are elevated.

5. Disorders in which there is a tendency of blood vessels to go into spasm such as angina pectoris, myocardial infarction, migraine, and Raynaud's syndrome; also hypertension.

6. Disorders of inflammation in which there may be excessive production of 2-series PG's from arachidonic acid, often coupled with low levels of cyclic AMP. Such disorders include multiple sclerosis, systemic lupus erythematosus, Crohn's disease; ulcerative colitis; inflammatory diseases of the kidney, for example, glomerulo-nephritis and nephrotic syndrome; inflammatory and degenerative diseases of the nervous and muscular systems, for example, muscular dystrophies, Friedreich's ataxia and related conditions of peripheral nerve degeneration; disorders of an auto-immune nature; and other collagen related diseases; rheumatoid arthritis and other inflammatory joint disorders; inflammatory skin disorders; disorders characterised by recurrent inflammation such as Familial Mediterranean Fever or Behcet's Syndrome.

Cancer details

One aspect of the present invention is in particular the provision of GLA or DGLA to by-pass the block at the delta-6-desaturase and enable cancer cells which have lost the enzyme to continue to make PGE1.

The connection between 1-series PG metabolism and reverse transformation is discussed more fully in the inventor's published EP—A—037 175. From this connection it would be expected that substances such as PGE1 might themselves induce the reverse transformation process. It is therefore significant that it is in fact known that PGE1 is able to induce reverse transformation in cultured cells, possibly through stimulating production of a nucleotide known as cyclic AMP (adenosine monophosphate) which is also known to induce the transformation. These reverse transformation inducing properties have been known since 1971, but the inventor believes that he is the first to recognise their current significance through his concern with prostaglandin metabolism generally. Through his approach, it has been possible to see that, while substances such as PGE1 and cyclic AMP would never be thought of as possible components of therapeutic compositions, being unstable and generally unsuitable for administration, restoration of the natural in situ production of 1-series PG's and in particular PGE1 is of great value.

The composition of the invention may thus be used in:—

A. A method of treating any condition in which enhancement of 1-series PG production, or more broadly influence of the 1-series/2-series PG balance in the body in favour of 1-series PG's, is indicated, and particularly, any of the conditions listed earlier herein, which comprises administering an effective amount of (a) γ-linolenic acid and/or dihomo-γ-linolenic acid (which may optionally be in association with linoleic and if desired other fat acids), said acids being used if desired as physiologically functional derivatives thereof, in conjunction with (b) an effective amount of glutathione.

B. A method of treating cancer in particular, which comprises administering an effective amount of (a) and (b) as last, preferably in conjunction with (c) an effective amount of thioproline.

Dose ranges

Dose ranges in humans, those for GLA and DGLA being given later, for example:

| | |
|---|---|
| Glutathione | 10 mg to 100 g/day, preferably 2 g to 20 g/day |
| Thioproline | 100 mg to 35 g/day, preferably 1 to 10 g.<br>A convenient specific amount is 40 mg/kilo body weight/day(ca3 g/day). |

Dose ranges for materials auxiliary to those of the invention are as discussed in the inventor's prior applications referred to herein. All the materials may be given in doses of for example one half, one third or one quarter of the above amounts.

Relationship to previous proposals

The above approach may be used in combination with the use of other materials as disclosed in the inventor's European Patent Applications.

These materials include zinc, penicillin and β-lactam antibiotics generally, EP—A—0 003 407) and also penicillamine, phenformin and levamisole (EP—A—0 004 770) when the other effects of these materials are acceptable, all of which are believed to enhance mobilisation of DGLA reserves and hence ensure that administered GLA and DGLA go into synthesis of PG's. The materials also include ascorbic acid, ethyl

alcohol, and naloxone, nalorphine, levallorphan and other opiate antagonists (EP—A—0 019 423), a class which enhance physiological synthesis of 1-series PGs from DGLA without substantially enhancing synthesis of 2-series PGs from AA.

Further, there is evidence that thromboxane A2 (produced in the body along with the endoperoxides giving rise to 2-series PGs) indirectly enhances formation of PGE1. Substances such as colchicine, amantadine, griseofulvin; vinblastine, vincristine and other Vinca alkaloids; interferon and melatonin, which are also discussed in the pending patent applications (EP—A—0 004 770) and which seem to increase production or action of thromboxane A2, are thus also desirably used in conjunction with the compositions of the present invention.

Reference may be made to the above published specifications for further details. The materials of the present invention may also be used in conjunction with the materials disclosed in published EP—A—0 035 856 namely chloroquine and other 4-aminoquinolines including amodiaquine and hydroxychloroquine; diidohydroxyquin and other 8-hydroxy and 8-aminoquinolines including iodochlorhydroxyquin, chiniofon, pentaquine, isopentaquine and primaquine; quinacrine (mepacrine) and other acridines; quinidine, quinine and procaine; emetine, metronidazole and other antiprotozoals; and spironolactone and other modified steroids, all of which influence the 1-series/2-series PG balance in the body in favour of 1-series PGs.

The purpose of these materials is thus not only to help in the restoration of 1-series PG production but to maintain a proper 1-series/2-series PG balance.

Packs

If it is not desired to have compositions comprising the active materials together, as listed above, packs may be prepared comprising the materials presented for separate or part joint and part separate administration in the appropriate relative amounts, and such packs are within the purview of the invention.

Dietary compositions

The invention is chiefly described in terms of pharmaceutical compositions, but it will be understood that the γ-linolenic and other acids, being in the nature of dietary supplements, could be incorporated in a dietary margarine or other foodstuffs; such foodstuffs, possibly containing other active materials and generally referred to in this description as dietary or pharmaceutical compositions, are within the purview of the invention and thus of the term pharmaceutical compositions, packs or the like.

Veterinary applications

It will be understood that where a disorder of a kind calling for treatment in animals arises, the invention while described primarily in terms of human medicine and treatment is equally applicable in the veterinary field.

Amounts of γ-linolenic and other acids specifically

A preferred daily dosage for all purposes for an adult (weight ca 75 kg) is from 0.05 to 0.1 up to 1, 2, 5 or even 10 g as required of γ-linolenic acid or equivalent weight calculated as γ-linolenic acid or a physiologically functional derivative thereof. Amounts in particular may be 0.1 to 1.0 g daily. Corresponding doses of the Oenothera oil discussed below containing 8 to 10% of γ-linolenic acid, are easily calculated. In place of, or in addition to, γ-linolenic acid, one may use dihomo-γ-linolenic acid or a physiologically functional derivative thereof, in amounts equivalent in molar terms to γ-linolenic acid and calculated as such. Other EFA's are likewise related back to γ-linolenic acid in molar terms. This dosage can for example be taken as a single dose or divided into 2, 3 or 4 subdivisions thereof as convenient.

Forms and sources of γ-linolenic and other acids

Suitable physiologically functional derivatives, convertible in the body to GLA or DGLA to enter the biosynthetic pathway given earlier herein, are physiologically acceptable salts, esters (particularly glycerides and simple $C_1$—$C_4$ alkyl esters), amides and phospholipids. Indirect identification of useful derivatives is by their having the valuable effect in the body of the acid (GLA or DGLA) itself, but conversion can be shown directly by gas chromatographic analysis of GLA or DGLA concentration in blood, body fat, or other tissue by standard techniques for example those of Pelick et al p. 23, "Analysis of Lipids and Lipoproteins" Ed. Perkins, American Oil Chemists Society, Champaign, Illinois, U.S.A. (1975) into compositions in the form of an available oil having a high γ-linolenic acid content, hence references to "oil" herein.

At the present time known natural sources of oils having a high γ-linolenic acid content are few (there are no known natural source of significant amounts of dihomo-γ-linolenic acid). One source of oils currently available is the seed of Evening Primrose species such as *Oenothera biennis L.* and *Oenothera lamarckiana,* the oil extract therefrom containing γ-linolenic acid (about 8%) and linoleic acid (about 72%) in the form of their glycerides together with other glycerides (percentages based on total fatty acids). Other sources of γ-linolenic acid are Borage species such as *Borago officinalis* which, though current yield per acre is low, provide a higher source of γ-linolenic acid than Oenothera oil. Recent studies on fungi which can be cultivated by fermentation promise a fungal oil source.

The seed oil extracts referred to above can be used as such or can for example if desired be fractionated to yield an oily composition containing the triglycerides of γ-linolenic and linoleic as the main fatty acid components, the γ-linolenic acid content being if desired a major proportion. Seed oil extracts appear to have a stabilising effect upon any dihomo-γ-linolenic acid or physiologically functional derivative thereof.

Pharmaceutical presentation

The compositions according to the invention are conveniently in a form suitable for oral, rectal, parenteral or topical administration in a suitable pharmaceutical vehicle, as discussed in detail for example in Williams GB—A—1 082 624, to which reference may be made, and in any case very well known generally for any particular kind of preparation. Thus for example tablets, capsules, ingestible liquid or powder preparations, creams and lotions for topical application, or suppositories, can be prepared as required. Injectable solutions of hydrolysed Oenothera oil may be prepared using albumin to solubilise the free acid.

Advantageously, a preservative is incorporated into the preparations. α-Tocopherol in a concentration of about 0.1% by weight has been found suitable for the purpose.

It will be understood that the absolute quantity of active ingredients present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following Examples serve to illustrate pharmaceutical compositions useful in treatment according to the invention:

Examples

Pharmaceutical compositions contain a unit dose of an oil extract from the seeds of *Oenothera biennis L.*, and of one of the active materials of the present invention, optionally with added DGLA for example as dihomo-γ-linolenate. They may be presented by encapsulation of the natural oil in soft gelatin capsules by known methods.

The oil is extracted from the seeds by one of the conventional methods of extraction such as cold pressure, screw pressure after partially cooking the seed, or solvent extraction.

Fractionation of a typical sample of this oil shows a yield of 97.0% in the form of methyl esters, with the relative proportions:

| | |
|---|---|
| Palmitate | 6.15 |
| Stearate | 1.6 |
| Oleate | 10.15 |
| Linoleate | 72.6 |
| γ-linolenate | 8.9 |

As preservative, α-tocopherol is added to the oil in a concentration of 0.1%.

Gelatin capsules containing oil extracts prepared as described above, each having the following contents of active ingredients (0.5 g oil extract=ca 0.045 g γ-linolenic acid), are prepared in conventional fashion.

The following Examples 1 and 2 give capsules that may be given in treatment of inflammatory disorders and the other conditions listed earlier, and generally in the treatment of conditions in which influence of the 1-series/2-series PG balance in the body if favour of 1-series PG's is indicated.

Example 1

Capsules containing:

0.5 g Evening Primrose oil
2 g glutathione

to be taken two capsules three times a day.

Example 2

Capsules to be taken as above, containing

0.5 g Evening Primrose oil
10 mg methyl dihomo-γ-linolenate
1 g glutathione

Example 3

Capsules as in Examples 1 and 2 but containing also thioproline 500 mg may be given specifically in the treatment of cancer.

**Claims for Contracting States: BE, CH, DE, FR, GB, IT, LI, LU, NL, SE**

1. A pharmaceutical composition comprising one or more essential fatty acids in conjunction with glutathione, the or each essential fatty acid being γ-linolenic acid or dihomo-γ-linolenic acid or physiologically functional derivative thereof convertible in the body to γ-linolenic acid or dihomo-γ-linolenic acid.

2. A composition according to claim 1 presented for administration to give 0.05 to 10 g of the γ-linolenic acid daily or molar equivalent of the dihomo-γ-linolenic acid or of the derivative.

3. A composition according to claim 1 or 2, presented for administration to give 10 mg to 100 g glutathione daily.

4. A composition according to claim 3, wherein said amount is 2 g to 20 g daily.

5. A composition according to any preceding claim comprising also thioproline.

6. A composition according to claim 5, presented for administration to give 100 mg to 35 g thioproline daily.

7. A composition according to claim 6, wherein said amount is 1 to 10 g daily.

8. A pharmaceutical pack comprising the materials set out in any preceding claim, presented separately, or one or more separately and others together, but for joint administration.

9. The composition of any of claims 1 to 4 or the pack of claim 8 as referring thereto for use in the treatment of any condition in which influence of the 1-series/2-series PG balance in the body in favour of 1-series PG's is indicated.

10. The composition of any of claims 1 to 4 or the pack of claim 8 as referring thereto for use in the treatment of inflammatory disorders.

11. The composition of any of claims 1 to 7 or the pack of claim 8 for use in the treatment of cancer.

12. The use of glutathione and of one or more essential fatty acids, the or each said essential fatty acid being as set out in claim 1, particularly in amounts as indicated in claim 2, 3 or 4, for the manufacture of a medicament for a therapeutic application as set out in claim 9 and 10.

13. The use according to claim 12 with the addition of thioproline, particularly in amounts as indicated in claim 6 or 7, for the manufacture of a medicament for a therapeutic application as set out in claim 11.

**Claims for Contracting State: AT**

1. The use of one or more essential fatty acids, in conjunction with glutathione and alone or in an acceptable pharmaceutical vehicle, the or each essential fatty acid being γ-linolenic acid or dihomo-γ-linolenic acid or physiologically function derivative thereof convertible in the body to γ-linolenic acid or dihomo-γ-linolenic acid for the method of providing a pharmaceutical composition for treatment of any condition in which influence of the 1-series/2-series PG balance in the body in favour of 1-series PGs is indicated, particularly inflammatory disorders or cancer.

2. Use according to claim 1 wherein said composition is presented for administration to give 0.05 to 10 g of the γ-linolenic acid daily or molar equivalent of the dihomo-γ-linolenic acid or of the derivative.

3. Use according to claim 1 or 2 wherein said composition is presented for administration to give 10 mg to 100 g glutathione daily.

4. Use according to claim 4, wherein said amount is 2 g to 20 g daily.

5. Use according to any preceding claim wherein said composition comprises also thioproline.

6. Use according to claim 5 wherein said composition is presented for administration to give 100 mg to 35 g thioproline daily.

7. Use according to claim 6, wherein said amount is 1 to 10 g daily.

**Patentansprüche für die Vertragsstaaten: BE, CH, DE, FR, GB, IT, LI, LU, NL, SE**

1. Pharmazeutische Zusammensetzung, bestehend aus mindestens einer essentiellen Fettsäure in Verbindung mit Glutathion, wobei die oder jede essentielle Fettsäure γ-Linolensäure oder Dihomo-γ-Linolensäure oder deren im Körper zu γ-Linolensäure oder Dihomo-γ-Linolensäure umwandelbares, physiologisch wirksames Derivat ist.

2. Zusammensetzung nach Anspruch 1 in einer Form, die die Verabreichung von 0,05 bis 10 g γ-Linolensäure täglich oder des molaren Äquivalents der Dihomo-γ-Linolensäure oder des Derivats ermöglicht.

3. Zusammensetzung nach Anspruch 1 oder 2 in einer Form, die die Verabreichung von 10 mg bis 100 g Glutathion täglich ermöglicht.

4. Zusammensetzung nach Anspruch 3, dadurch gekennzeichnet, daß die erwähnte Menge 2 g bis 20 g täglich beträgt.

5. Zusammensetzung nach einem der vorhergehenden Ansprüche mit zusätzlichem Thioprolin.

6. Zusammensetzung nach Anspruch 5 in einer Form, die die Verabreichung von 100 mg bis 35 g Thioprolin täglich ermöglicht.

7. Zusammensetzung nach Anspruch 6, dadurch gekennzeichnet, daß die erwähnte Menge 1 bis 10 g täglich beträgt.

8. Pharmazeutische Packung, bestehend aus den in einem der vorhergehenden Ansprüche angegebenen Substanzen, jeweils getrennt oder mindestens eine Substanz getrennt und andere Substanzen in Verbindung miteinander, jedoch zur gemeinsamen Verabreichung.

9. Zusammensetzung nach einem der Ansprüche 1 bis 4 oder die darauf bezogene Packung nach Anspruch 8, zur Verwendung in der Behandlung eines Zustands, bei welchem die Beeinflussung des Verhältnisses Prostaglandine 1.Serie/Prostaglandin 2.Serie im Körper zugunsten von Prostaglandinen 1.Serie indiziert ist.

10. Zusammensetzung nach einem der Ansprüche 1 bis 4 oder die darauf bezogene Packung nach Anspruch 8, zur Behandlung von Entzündungskrankheiten.

11. Zusammensetzung nach einem der Ansprüche 1 bis 7 oder die Packung nach Anspruch 8, zur Krebsbehandlung.

12. Verwendung von Glutathion und mindestens einer der in Anspruch 1 angegebenen essentiellen Fettsäuren, insbesondere in den in den Ansprüchen 2, 3 oder 4 angegebenen Mengen, zur Herstellung eines Medikaments für eine therapeutische Anwendung der in den Ansprüchen 9 und 10 angegebenen Art.

13. Verwendung nach Anspruch 12, unter Zusatz von Thioprolin, insbesondere in den in den Ansprüchen 6 und 7 angegebenen Mengen, zur Herstellung eines Medikaments für eine therapeutische Anwendung gemäß Anspruch 11.

**Patentansprüche für den Vertragsstaat: AT**

1. Verwendung von mindestens einer essentiellen Fettsäure in Verbindung mit Glutathion, jeweils allein oder in einem geeigneten pharmazeutischen Träger, wobei die oder jede essentielle Fettsäure γ-Linolensäure oder Dihomo-γ-Linolensäure oder deren im Körper zu γ-Linolensäure oder Dihomo-γ-Linolensäure umwandelbares, physiologisch wirksames Derivat ist, für das Verfahren zur Bildung einer pharmazeutischen Zusammensetzung für die Behandlung eines Zustands, bei dem die Beeinflussung des Verhältnisses Prostaglandine 1.Serie/Prostaglandine 2.Serie im Körper zugunsten von Prostaglandinen 1.Serie indiziert ist, insbesondere von Entzündungskrankheiten oder Krebs.

2. Verwendung nach Anspruch 1, dadurch gekennzeichnet, daß die Zusammensetzung in einer Form dargeboten wird, die die Verabreichung von 0,05 bis 10 g γ-Linolensäure täglich oder des molaren Äquivalents der Dihomo-γ-Linolensäure oder des Derivats ermöglicht.

3. Verwendung nach Anspruch 1 oder 2, dadurch gekennzeichnet, daß die Zusammensetzung in einer Form dargeboten wird, die die Verabreichung von 10 mg bis 100 g Glutathion täglich ermöglicht.

4. Verwendung nach Anspruch 3, dadurch gekennzeichnet, daß die erwähnte Menge 2 g bis 20 g täglich beträgt.

5. Verwendung nach einem der vorhergehenden Ansprüche, dadurch gekennzeichnet, daß die Zusammensetzung zusätzlich Thioprolin enthält.

6. Verwendung nach Anspruch 5, dadurch gekennzeichnet, daß die Zusammensetzung in einer Form dargeboten wird, die die Verabreichung von 100 mg bis 35 g Thioprolin täglich ermöglicht.

7. Verwendung nach Anspruch 6, dadurch gekennzeichnet, daß die erwähnte Menge 1 bis 10 g täglich beträgt.

**Revendications pour les Etats Contractants: BE, CH, DE, FR, GB, IT, LI, NL, SE, LU**

1. Une composition pharmaceutique comprenant un ou plusieurs acides gras essentiels en association avec du glutathion, l'acide essentiel ou chacun des acides essentiels étant l'acide γ-linolénique ou l'acide dihomo-γ-linolénique ou un dérivé physiologiquement fonctionnel de ceux-ci convertible dans l'organisme en acide γ-linolénique ou en acide dihomo-γ-linolénique.

2. Une composition selon la revendication 1 présentée pour l'administration pour apporter 0,05 à 10 g d'acide γ-linolénique par jour ou l'équivalent molaire de l'acide dihomo-γ-linolénique ou du dérivé.

3. Une composition selon la revendication 1 ou 2 présentée pour l'administration pour apporter 10 mg à 100 mg de glutathion par jour.

4. Une composition selon la revendication 3, dans laquelle ladite quantité est de 2 g à 20 g par jour.

5. Une composition selon l'une quelconque des revendications précédentes comprenant également de la thioproline.

6. Une composition selon la revendication 5 présentée pour l'administration pour apporter 100 mg à 35 g de thioproline par jour.

7. Une composition selon la revendication 6, dans laquelle ladite quantité est de 1 à 10 g par jour.

8. Un coffret pharmaceutique comprenant les matières indiquées dans l'une quelconque des revendications précédentes, présentées séparément ou une ou plusieurs séparément et les autres ensemble, mais pour l'administration conjointe.

9. La composition de l'une quelconque des revendications 1 à 4 ou le coffret de la revendication 8 s'y rapportant pour l'emploi dans le traitement de tout état dans lequel il est indiqué d'influer sur l'équilibre PG de la série 1/PG de la série 2 dans l'organisme en faveur des PG de la série 1.

10. La composition selon l'une quelconque des revendications 1 à 4 ou le coffret de la revendication 8 s'y rapportant pour l'emploi dans le traitement de troubles inflammatoires.

11. La composition de l'une quelconque des revendications 1 à 7 ou le coffret de la revendication 8 pour l'emploi dans le traitement du cancer.

12. L'emploi de glutathion et d'un ou plusieurs acides gras essentiels, ledit ou chacun desdits acides gras essentiels étant indiqué dans la revendication 1, en particulier en les quantités indiquées dans les revendications 2, 3 ou 4, pour la fabrication d'un médicament pour une application thérapeutique comme indiqué dans les revendications 9 et 10.

13. L'emploi selon la revendication 12 avec l'addition de thioproline, en particulier en les quantités indiquées dans les revendications 6 ou 7 pour la fabrication d'un médicament pour une application thérapeutique comme indiqué dans la revendication 11.

**Revendications pour l'Etat Contractant: AT**

1. L'emploi d'un ou plusieurs acides gras essentiels en association avec du glutathion et isolément ou dans un véhicule pharmaceutique acceptable, l'acide gras essentiel ou chaque acide gras essentiel étant l'acide γ-linolénique ou l'acide dihomo-γ-linolénique ou un dérivé physiologiquement fonctionnel de ceux-ci convertible dans l'organisme en acide γ-linolénique ou en acide dihomo-γ-linolénique pour le procédé de fourniture d'une composition pharmaceutique pour le traitement de tout état dans lequel il est indiqué d'influer sur l'équilibre PG de la série 1/PG de la série 2 dans l'organisme en faveur des PG de la série 1, en particulier les troubles inflammatoires ou le cancer.

2. Emploi selon la revendication 1, dans lequel ladite composition est présentée pour l'administration pour apporter 0,05 à 10 g de l'acide γ-linolénique par jour ou l'équivalent molaire de l'acide dihomo-γ-linolénique ou du dérivé.

3. Emploi selon la revendication 1 ou 2, dans lequel ladite composition est présentée pour l'administration pour apporter 10 mg à 100 g de glutathion par jour.

4. Emploi selon la revendication 4 dans lequel ladite quantité est de 2 g à 20 g par jour.

5. Emploi selon l'une quelconque des revendications précédentes dans lequel ladite composition comprend également de la thioproline.

6. Emploi selon la revendication 5, dans lequel ladite composition est présentée pour l'administration pour apporter 100 mg à 35 g de thioproline par jour.

7. Emploi selon la revendication 6, dans lequel ladite quantité est de 1 à 10 g par jour.